# EUROPEAN PATENT APPLICATION

(11) **EP 2 100 626 A1**
(43) Date of publication of application: **16.09.2009**
(21) Application number: 09250693.0
(22) Date of filing: 13.03.2009
(51) Int. Cl.: A61L 9/16, B01D 53/00, B01J 23/30, B01J 35/10, B01J 37/00

(54) **Process for decomposing volatile aromatic compound**

(30) Priority: 13.03.2008 JP 2008063755
(71) Applicant: Sumitomo Chemical Company, Limited, Tokyo 104-8260 (JP)
(72) Inventor: Sakatani, Yoshiaki, Niihama-shi, Ehime 792-0025 (JP)
(74) Representative: Duckworth, Timothy John

(57) **Abstract**

A process for decomposing a volatile aromatic compound in the vapor phase, which process comprises:
a step of bringing the volatile aromatic compound into contact, under light irradiation, with a photocatalyst layer formed by applying a photocatalyst dispersion liquid to a substrate,

wherein the photocatalyst dispersion liquid comprises titanium oxide photocatalyst particles and tungsten oxide photocatalyst particles dispersed in a dispersion medium, and the surfaces of the titanium oxide photocatalyst particles are charged in the same polarity as the surfaces of the tungsten oxide photocatalyst particles.

## Description

### BACKGROUND OF THE INVENTION

### 1. Field of the Invention

The present invention relates to a process for decomposing a volatile aromatic compound and, more particularly, a process for bringing an aromatic compound contained in a vapor phase into contact with a photocatalyst layer under light irradiation so as to decompose the aromatic compound.

### 2. Description of Related art

When light having energy higher than the band cap is irradiated to a semiconductor, electrons in a valence band are excited to a conduction band so that positive holes are generated in the valence band. The excited electrons and the positive holes respectively have strong reducing energy and oxidizing energy, and thus exert a reduction and oxidation reaction on molecular species in contact with the semiconductor. This reduction and oxidation reaction is called a photocatalytic reaction, and a semiconductor which enables to have such the photocatalytic activity is called a photocatalyst. As for such the photocatalyst, a particle-like photocatalyst such as titanium oxide photocatalyst particles and tungsten oxide photocatalyst particles is known.

A photocatalyst dispersion liquid obtained by dispersing such the titanium oxide photocatalyst particles and tungsten oxide photocatalyst particles in a dispersion medium is known (Japanese Patent Application Laid-Open No. 2005-231935). By coating this photocatalyst dispersion liquid on a surface of a substrate, a photocatalyst layer containing titanium oxide photocatalyst particles and tungsten oxide photocatalyst particles and having a photocatalytic activity can be easily formed on the surface of the substrate, and a volatile organic compound contained in a vapor phase can be decomposed by the photocatalyst layer.

### BRIEF SUMMARY OF THE INVENTION

However, the convention process using the photocatalyst layer formed from the photocatalyst dispersion liquid has a problem that it cannot swiftly decompose the aromatic compound in a vapor phase.

Present inventors carried out earnest works so as to develop a process capable of swiftly decomposing an aromatic compound in a vapor phase. As a result, they completed the present invention.

That is, the present invention is to provide a process for decomposing a volatile aromatic compound including the step of bringing a volatile aromatic compound contained in a vapor phase into contact with a following photocatalyst layer under light irradiation. The photocatalyst layer:
The photocatalyst layer is formed by coating a photocatalyst dispersion liquid on a substrate. The photocatalyst dispersion liquid is obtained by dispersing titanium oxide photocatalyst particles and tungsten oxide photocatalyst particles in a dispersion medium, and the surfaces of the titanium oxide photocatalyst particles are charged in the same polarity as the surfaces of the tungsten oxide photocatalyst particles are.
According to the decomposing process of the present invention, a volatile aromatic compound in a vapor phase can be decomposed swiftly.
The above and further objects and features of the invention will more fully be apparent from the following detailed description.

### DETAILED DESCRIPTION OF THE INVENTION

A photocatalyst layer used in the decomposing process of the present invention is formed by coating a photocatalyst dispersion liquid on a substrate. The photocatalyst dispersion liquid is obtained by dispersing titanium oxide photocatalyst particles and tungsten oxide photocatalyst particles in a dispersion medium, and the surfaces of the titanium oxide photocatalyst particles are charged in the same polarity as the surfaces of the tungsten oxide photocatalyst particles are.

### [Titanium oxide photocatalyst particles]

The titanium oxide photocatalyst particles composing a photocatalyst dispersion liquid are particle-like titanium oxide having a photocatalytic activity and, for example, meta-titanic acid particles or titanium dioxide (TiO₂) particles in which a crystal structure is an anatase type, brookite type or rutile type.

For example, the meta-titanic acid particles can be obtained by the following process 1.

Process 1: Process for hydrolyzing a titanyl sulfate aqueous solution with heating.

For example, the titanium dioxide particles can be obtained by any one process of the following processes 2-1 to 2-3.

Process 2-1: Process for adding a base to a titanyl sulfate or titanium chloride aqueous solution without heating so as to obtain a precipitate, and calcining the precipitate.

Process 2-2: Process for adding water, an acid aqueous solution, or a basic aqueous solution to a titanium alkoxide so as to obtain a precipitate, and calcining the precipitate.

Process 2-3: Process for calcining meta-titanic acid. The titanium dioxide particles obtained by these processes 2-1 to 2-3 can be obtained as anatase-type, brookite-type or rutile-type particles depending on a calcining temperature and a calcining time at a time of calcining.

As for the particle diameters of the titanium oxide photocatalyst particles, the average dispersed particle diameter is generally from 20nm to 150nm, preferably from 40nm to 100nm, from a view point of a photocatalytic activity.

The BET specific surface of the titanium oxide photocatalyst particles is generally from 100 m²/g to 500 m²/g, preferably from 300 m²/g to 400 m²/g, from the view point of a photocatalytic activity.

### [Tungsten oxide photocatalyst particles]

The tungsten oxide photocatalyst particles are particle-like tungsten oxide having a photocatalytic activity, and tungsten trioxide (WO₃) particles are used in general. For example, the tungsten trioxide particles can be obtained by a process for adding an acid to a tungstate aqueous solution so as to obtain tungstic acid as a precipitate, and calcining the tungstic acid. Further, the tungsten trioxide particles can be also obtained by a process for thermally decomposing ammonium metatungstate or ammonium paratungstate with heating.

As for the particle diameters of the tungsten oxide photocatalyst particles, an average dispersed particle diameter is generally from 50nm to 200nm, preferably from 80nm to 130nm, from the view point of a photocatalytic activity.

The BET specific surface of the tungsten oxide photocatalyst particles is generally from 5 m²/g to 100 m²/g, preferably from 20 m²/g to 50 m²/g, from the view point of a photocatalytic activity.

The mass ratio of use amounts of titanium oxide photocatalyst particles and tungsten oxide photocatalyst particles is generally from 4:1 to 1:8, and preferably from 2:3 to 3:2.

### [Dispersion medium]

As for the dispersion medium, a water medium mainly containing water is used. More particularly, a medium containing a use amount of water equal to or greater than 50% by mass is used. Water can be used independently, or a mixed solvent of water and a water-soluble organic solvent can be used. As for the water-soluble organic solvent, for example, a water-soluble alcoholic solvent such as methanol, ethanol, propanol or butanol, acetone, methylethyl ketone, and the like can be used.

A use amount of the dispersion medium is generally from 5 mass times to 200 mass times, preferably from 10 mass times to 100 mass times, with respect to a total amount of the titanium oxide photocatalyst particles and the tungsten oxide photocatalyst particles. When the use amount of the dispersion medium is less than 5 mass times, the titanium oxide photocatalyst particles and the tungsten oxide photocatalyst particles are precipitated easily. When the use amount is more than 200 mass times, there is disadvantageous in the view point of volume efficiency.

### [Photocatalyst dispersion liquid]

The hydrogen ion concentration of the photocatalyst dispersion liquid is generally from pH 0.5 to pH 8.0, and preferably from pH 1.0 to pH 7.0. When the hydrogen ion concentration has pH of smaller than 0.5, acidity is too strong, and thus the liquid is hardly handled. When the hydrogen ion concentration has pH of greater than 8.0, the tungsten oxide photocatalyst particles may be dissolved. The hydrogen ion concentration of the photocatalyst dispersion liquid can be generally adjusted by adding an acid. As for the acid, for example, nitric acid, hydrochloric acid, sulfuric acid, phosphoric acid, formic acid, acetic acid, oxalic acid, or the like can be used.

### [Electrification of a surface]

In the photocatalyst dispersion liquid, the surfaces of the titanium oxide photocatalyst particles and the tungsten oxide photocatalyst particles are charged in the same polarity. More particularly, both the surfaces are charged positively or negatively.

The surfaces of the meta-titanic acid particles obtained by the above-described process 1 and the titanium dioxide particles obtained by the above-described processes 2-1 to 2-3 are charged positively in general.

On the other hand, as for the tungsten oxide particles obtained by the process for adding an acid to a tungstate aqueous solution so as to obtain tungstic acid as a precipitate and calcining the tungstic acid, and the tungsten oxide particles obtained by thermally decomposing ammonium metatungstate and ammonium paratungstate with heating, the surfaces of these particles are charged negatively.

Therefore, when the titanium oxide photocatalyst particles having the positively charged surfaces and tungsten oxide photocatalyst particles having the negatively charged surfaces are used, for example, the surfaces of the titanium oxide photocatalyst particles are made to be charged negatively so as to be used in the photocatalyst dispersion liquid of the present invention.

In order to make the positively charged surfaces of the titanium oxide photocatalyst particles to the negatively charged surfaces, the titanium oxide photocatalyst particles can be dispersed in a solution in which a surface treatment agent for making the surfaces of the titanium oxide photocatalyst particles to charge negatively is dissolved in the dispersion medium. As for such the surface treatment agent, for example, polycarboxylic acid such as dicarboxylic acid or tricarboxylic acid, or phosphoric acid can be used. For example, oxalic acid or the like can be used as the dicarboxylic acid, and citric acid or the like can be used as the tricarboxylic acid. A free acid or a salt can be used as polycarboxylic acid and phosphoric acid. As for a salt, for example, an ammonium salt or the like can be used. As for the surface treatment agent, oxalic acid, ammonium oxalate or the like can be used preferably.

The use amount of the surface treatment agent is generally 0.001 mol times or more, preferably 0.02 mol times or more, in order to sufficiently charge the surfaces of the titanium oxide photocatalyst particles in terms of TiO₂. The use amount of the surface treatment agent is generally 0.5 mol times or less, preferably 0.3 mol times or less, from the view point of economical efficiency.

By dispersing the titanium oxide photocatalyst particles having the positively charged surfaces in the solution in which the surface treatment agent is dissolved with the dispersion medium, the surface treatment agent dissolved in the surface treatment solution is adsorbed on the surfaces of the titanium oxide photocatalyst particles, and thereby the surfaces can be charged negatively.

The electrifications of surfaces of the titanium oxide photocatalyst particles and the tungsten oxide photocatalyst particles can be measured by a zeta potential at the time of respectively dispersing the particles in a solvent. As for the solvent used for measuring the zeta potential, a sodium chloride aqueous solution (having a sodium chloride concentration of 0.01 mol/L), which is added with hydrochloric acid to have a hydrogen ion concentration having pH 3.0, is used. The use amount of the solvent is generally 10000 mass times to 1000000 mass times with respect to the titanium oxide photocatalyst particles or tungsten oxide photocatalyst particles.

### [Producing of a photocatalyst dispersion liquid]

The photocatalyst dispersion liquid of the present invention can be obtained by dispersing the titanium oxide photocatalyst particles having the positively charged surfaces in the solution in which the surface treatment agent is dissolved with the dispersion medium, and mixing the particles with the tungsten oxide photocatalyst particles having the negatively charged surfaces.

The photocatalyst dispersion liquid can be also obtained by dispersing the titanium oxide photocatalyst particles having the positively charged surfaces in the above-described solution, subjecting the particles to a dispersion treatment, and mixing those with the tungsten oxide photocatalyst particles having the negatively charged surfaces. For example, the dispersion treatment can be carried out by a general method using a medium stirring dispersion machine.

Although the tungsten oxide photocatalyst particles can be mixed as they are, these particles are generally mixed in a state of being dispersed in the dispersion medium, and preferably mixed after subjecting to a dispersion treatment. For example, the dispersion treatment can be carried out by a general method using a medium stirring dispersion machine.

### [Electron-withdrawing substance or its precursor]

The photocatalyst dispersion liquid can contain an electron-withdrawing substance or its precursor. The electron-withdrawing substance is supported on the surface of the photocatalyst so as to exert electron-withdrawing property. The photocatalytic activity can be more increased by supporting the electron-withdrawing substance on the surface of the photocatalyst and thereby suppressing the recombination of electrons and positive holes, where the electrons are excited at the conduction band and the positive holes are generated at the valence band by irradiation of light.

As for such the electron-withdrawing substance, for example, metals such as Cu, Pt, Au, Pd, Ag, Fe, Nb, Ru, Ir, Rh, Co and the like, preferably Cu, Pt, Au, Pd can be used. Further, oxides and hydroxides of these metals can be also used. The electron-withdrawing substance is generally dispersed in a photocatalyst dispersion liquid as colloid particles.

The precursor of the electron-withdrawing substance is a compound that can convert to an electron-withdrawing substance on the surface of a photocatalyst. For example, such the precursor is nitrate, sulfate, halide, an organic acid salt, carbonate, phosphate or the like of the above-described metal. More particularly, for example, as for a precursor of copper, copper nitrate (Cu(NO₃)₂), copper sulfate (CuSO₄), copper chloride (CuCl₂, CuCl), copper bromide (CuBr₂, CuBr), copper iodide (CuI), copper iodate (CuI₂, O₆), copper ammonium chloride (Cu(NH₄)₂Cl₄), copper oxychloride (Cu₂Cl(OH)₃), copper acetate (CH₃COOCu, (CH₃COO)₂Cu), copper formate ((HCOO) ₂Cu), copper carbonate (CuCO₃), copper oxalate (CuC₂O₄), copper citrate (Cu₂C₆H₄O₇), copper phosphate (CuPO₄), or the like, can be used. As for a precursor of platinum, platinum chloride (PtCl₂, PtCl₄), platinum bromide (PtBr₂, PtBr₄), platinum iodide (PtI₂, PtI₄), potassium platinum chloride (K₂(PtCl₄)), hexachloroplatinic acid (H₂PtCl₆), platinum sulfite (H₃(Pt(SO₃)₂OH), platinum oxide (PtO₂), tetrammine platinum chloride (Pt(NH₃)₄Cl₂), tetrammine platinum hydrogencarbonate (C₂H₁₄N₄O₆Pt), tetrammine platinum hydrogenphosphate (Pt(NH₃)₄HPO₄), tetrammine platinum hydroxide (Pt(NH₃)₄(OH)₂), tetrmmine platinum nitrate (Pt(NO₃)₂(NH₃)₄), tetrmmine platinum tetrachloroplatinum ((Pt(NH₃)₄)(PtCl₄)), or the like, can be used.

As for a precursor of Au, gold chloride (AuCl), gold bromide (AuBr), gold iodide (AuI), gold hydroxide, (Au (OH)₂), tetrachlorochloroauric acid (HAuCl₄), potassium tetrachlorochloroaurate (KAuCl₄), potassium tetrabromochloroaurate (KAuBr₄), gold oxide (Au₂O₃), or the like, can be used. As for a precursor of palladium, palladium acetate ((CH₃COO)₂Pd), palladium chloride (PdCl₂), palladium bromide (PdBr₂), palladium iodide (PdI₂), palladium hydroxide (Pd(OH)₂), palladium nitrate (Pd(NO₃)₂), palladium oxide (PdO), palladium sulfate (PdSO₄), potassium tetrachloropalladium acid (K₂(PdCl₄)), potassium tetrabromopalladium acid (K₂(PdBr₄)), or the like, can be used.

These electron-withdrawing substances or these precursors can be used independently or by mixing two or more kinds.

When the electron-withdrawing substance or its precursor is used, the use amount is generally from 0.005 parts by mass to 0.6 parts by mass, preferably from 0.01 parts by mass to 0.4 parts by mass, in terms of the metal atom with respect to the total amount of 100 parts by mass of the titanium oxide photocatalyst particles and the tungsten oxide photocatalyst particles. When the use amount is less than 0.005 parts by mass, the photocatalytic activity by the use of the electron-withdrawing substance is not sufficiently improved. When the use amount is more than 0.6 parts by mass, the photocatalytic activity is to be insufficient easily.

For example, the photocatalytst dispersion liquid containing the electron-withdrawing substance or its precursor can be obtained by the similar process to that described above, that is, mixing the titanium oxide photocatalyst dispersion liquid and the tungsten oxide photocatalyst dispersion liquid, and adding the electron-withdrawing substance or its precursor to the mixture. When the precursor is added, light-irradiation can be carried out after the addition. A light to be irradiated is a visible radiation or an ultraviolet radiation. By carrying out the light-irradiation, the precursor can be converted to the electron-withdrawing substance. When light having the light-excitable wavelength of the photocatalyst is irradiated, electrons are generated by the light excitation so as to reduce the precursor, and then the precursor is supported by the surfaces of the photocatalyst particles as the electron-withdrawing substance.

### [Additive]

The photocatalyst dispersion liquid of the present invention can be added with an additive within the range not change the electrifications of the titanium oxide photocatalyst particles and the tungsten oxide photocatalyst particles.

As for the additive, for example, a material added for improving the photocatalytic activity can be used. More particularly, the additive is a silicon compound such as amorphous silica, silica sol, liquid glass, organopolysiloxane or the like, an aluminum compound such as amorphous alumina, alumina sol, an aluminum hydroxide or the like, an aluminosilicate such as zeolite, kaolinite or the like, an alkali earth metal oxide or an alkali earth metal hydroxide such as magnesium oxide, calcium oxide, strontium oxide, barium oxide, magnesium hydroxide, calcium hydroxide, strontium hydroxide, barium hydroxide or the like, calcium phosphate, molecular sieve, active carbon, a polycondensation product of an organopolysiloxane compound, phosphate, a fluorine-based polymer, a silicon-based polymer, an acrylic resin, a polyester resin, a melamine resin, an urethane resin, an alkyd resin, or the like. The additive can be used independently or by mixing two or more kinds.

Further, a binder can be used, and this binder is for more strongly holding the titanium oxide photocatalyst particles and the tungsten oxide photocatalyst particles on the surface of a substrate when coating the photocatalyst dispersion liquid on the surface of the substrate (Japanese Patent Application Laid-Open No. 8-67835, Japanese Patent Application Laid-Open No. 9-25437, Japanese Patent Application Laid-Open No.10-183061, Japanese Patent Application Laid-Open No.10-183062, Japanese Patent Application Laid-Open No.10-168349, Japanese Patent Application Laid-Open No.10-225658, Japanese Patent Application Laid-Open No. 11-1620, Japanese Patent Application Laid-Open No.11-1661, Japanese Patent Application Laid-Open No.2004-59686, Japanese Patent Application Laid-Open No.2004-107381, Japanese Patent Application Laid-Open No.2004-256590, Japanese Patent Application Laid-Open No.2004-359902, Japanese Patent Application Laid-Open No.2005-113028, Japanese Patent Application Laid-Open No.2005-230661, Japanese Patent Application Laid-Open No. 2007-161824).

For example, the photocatalyst dispersion liquid containing the additive can be obtained by a process adding the additive to the photocatalyst dispersion liquid which is obtained by mixing the titanium oxide photocatalyst dispersion liquid and the tungsten oxide photocatalyst dispersion liquid.

### [Forming of a photocatalyst layer]

A photocatalyst functional product can be produced by coating the photocatalyst dispersion liquid on the surface of a substrate, and volatilizing the dispersion medium, where the product has a photocatalyst layer, on the surface of the substrate, containing the titanium oxide photocatalyst particles and the tungsten oxide photocatalyst particles so as to indicate a photocatalytic activity.

When the photocatalyst dispersion liquid contains the electron-withdrawing substance or its precursor, the electron-withdrawing substance or its precursor is supported by the surfaces of the titanium oxide photocatalyst particles and the tungsten oxide photocatalyst particles. When the precursor is used, the supported precursor is converted to the electron-withdrawing substance after supporting.

### [Photocatalyst functional products]

The photocatalyst functional product generally holds the photocatalyst on the surface with the strength that can be equal to practical use. As for the shape and size the photocatalyst used in this case, various kinds of shapes such as a particle, fiber, a thin piece and the like can be applied and a size can be properly selected corresponding to an application and surface property. Further, when the photocatalyst is formed as a film on the surface of the photocatalyst functional product, the film thickness can be properly selected and formed to be several hundred nm to several mm. The photocatalyst is preferably held on the surface to which a visible radiation is irradiated and which is continuously or intermittently, and spatially connected with a part generating a malodorous substance, among the inner surface and outer surface of the photocatalyst functional product made of a material such as plastics, a metal, ceramics, wood, concrete or paper.

As for examples of the photocatalyst functional products, the followings are described.

That is, clothes (underwear, nightclothes, western clothes, Japanese clothes, an apron), furnishings (socks, a hat, a tie, a handkerchief, a belt), personal effects (an umbrella, a stick, a folding fan, a round fan, an accessory, eyeglasses, a wig), a bag, a portable bag, shoes, smoker's requisites (a smoking pipe, a smoking pipe rest, a smoking pipe cleaning tool, a tobacco pipe, a cigarette pouch, an ashtray, a lighter, a table lighter, a lighter with a watch, a matchbox, a gas igniter), a cosmetic and hairdressing tools (a compact, a perfume bottle, an atomizer, a pocket mirror, a hand mirror, a shaving brush, a brush for makeup, a comb, a hair brush, a nail clipper, a hair roller, a hair iron, a hair drier, a knife for manicure, a cuticle pusher for manicure, a nail file, a hair clipper, an electric hair clipper, a western razor, a Japanese razor, a safety razor, a spare razor blade, a handy razor, an electric razor, a chair for hairdressing, a button, a zipper), rugs (a carpet, a tatami facing, an edge materials of a tatami, a flower straw mat, a cushion, a cushion cover, a floor cushion, a floor cushion cover, a bath mat, a doormat, an electric carpet), bedclothes (a futon, a futon cover, a blanket, a bed sheet, a pillow, a pillow cover, a sleeping-bag, a mattress), a curtain, a Venetian blind, a slat for Venetian blinds, the bottom rail for Venetian blinds, a ladder tape for Venetian blinds, a blind, a shop curtain, a beads shop curtain, a tablecloth, a napkin, a centerpiece, a place mat, room decorations (a vase, an ornament, a frame, a pole hanging, a wall hanging, a tablet tray, an artificial flower), a wallpaper, a deodorant, washing and a cleaning equipments (a tub, a washboard, an electrical washing machine, an electric washing machine with a clothes dryer, a drain hose for electrical washing machines, a clothespole, a clothespole supporter, a clothespole prop, a laundry hanger, a washing string, a clothespin, a clothes dryer, a futon dryer, an iron, an iron placing stand, an ironing board, a trouser press, a washing finishing machine, a broom, a brush for cleaning, a dress brush, a dustpan, a duster, a dustcloth, a mop, a handy cleaner, a garbage can, a vacuum cleaner, an electric floor polishing machine, a brush for electric floor polishing machine, an electric shoeshine machine), a home sanitation supplies (a toothbrush, a toothbrush case, a toothbrush stand, an electric toothbrush, the brush for electric toothbrush, tweezers, an earpick, a dirt remover, a brush for bathing, a soap box, a washbasin, a washcloth, a towel, an eyedropper, an eye washing apparatus, an electric mosquito-repelling device, an insecticide container, a deodorizer container), containers for cooking, eating and drinking (a washtub, a dish drainer, a rice washer, a pickles machine, an iron pot, a rice cooker, an electronic rice warmer, a pan, a boiled egg maker, a pot lid, a knob for pot lid, a drop cover for pot, a handle for pot, a kettle, an electric kettle, a sake-heating device, a steamer, a bamboo steamer, a frying pan, an egg fryer, a coffee maker, a cooking grid, a grill plate), cooking utensils (a cooking stove, a range, a microwave oven, an oven, a toaster, a toaster oven, a roaster, an electromagnetic induction cooker, a peeler, a corer for fruits, a grater, a home meat grinder, a slicer, an ice shaver, a cutter for dried-bonito-flakes, a juicer mixer, a juicer, a lemon squeezer, a food mixer, a hand mixer, a whisk, a cocktail shaker, an ice cream maker, a coffee grinder, a spice grinder, a sesame pounding machine, an ice pick, a nutcracker, a noodle making machine for home use, a rice cake making machine, a strainer for cooking, a tea strainer, a dripper, a squeezer for cooking, a can opener, a corkscrew, a sushi molding machine, a molding machine for confectionery products, an ice tray, a handle for kitchen knives, scissors for cooking, a cheese cutter, an egg cutter, a dish washer, a dish dryer, a heat preserving chamber, a refrigerator, a freezer, an ice machine, a water cooler, a dispenser for beverage, a measuring rice tub, a home water purifier, a glass stand, a plate rack, a kitchen knife rack, a glass holder, a bottle stand, an alcohol cradle, a tray, a small diner table, a saucer, a glass holder, a pot stand, a dish stand, a glass thermal cover, a covering for toasters, a napkin holder, a napkin ring, a caster stand, a chopstick case, a chopstick rest, a chopstick holder, a toothpick holder), congratulations-and-condolences goods (a household Shinto altar, an offertory box, a box for fortune slips, an icon, a Buddhist altar, a decoration implement for canopy, a Buddha statue, a rosary, a sutra desk, a bell for Buddhist services, an altar, a gravestone, a grave marker, a coffin, a flower vase for funerals and festivals, a censer for funerals and festivals, a light for funerals and festivals, a candlestick for funerals and festivals, a small offering stand, a talisman, a charm bag, a portable shrine, a bag for congratulation money, a bag for condolence money, a gift wrapping paper, a ceremonial paper string, a Christmas tree, an ornament for Christmas tree, a braid for Christmas), household goods (a sewing spatula, a tailors chalk, a bobbin for sawing, a sawing needle, a pincushion, a sewing box, a thimble, a knitting needle, a jewel box, a ring case, a home sprayer, a nozzle of a home sprayer), furniture (a bed, a hammock, a chair, a bench, a sofa bed, a legless chair, a chair covering, a seat, an outdoor bench, a leg for chairs, an infant walker, a desk, a desk with a chair, a bookrest, a low table, a table, a table leg, a counter, a television rack, a plant stand, a service wagon, a wardrobe, a cabinet, a corner cabinet, a bookshelf, a sideboard, a cupboard, a bureau, a hanging cupboard, a shoe cupboard, a sorting box for clothes, a lacquer low box, a cabinet for commodities, a safe, a portable cashbox, a dial lock for safes, a locker, a clothes-changing basket, a dresser, a full-length mirror, a wall-mounted mirror, a desk stand mirror, a screen, a folding screen, a hat rack stand, a hinge for furniture, a lock for furniture, a pull for furniture, a handle for furniture, a knob for furniture, a shelf receiver for furniture, a shelf board for furniture, a decorative metal fitting for furniture, a door stop for furniture), indoor small arranging boxes (a hook for clothing, a hanger for clothing, a hanger for skirts, a necktie hanger, a towel rail, a duster rail, a hook for hanger boards, a magazine rack, a newspapers rack, an umbrella stand, a slippers stand, a under floor storage), lighting fixtures (an incandescent lamp, an incandescent lamp for ornaments, a sealed beam lamp, an infrared lamp, a halogen lamp, a fluorescent lamp, a glow starter, a sodium lamp, a xenon lamp, a ceiling light, a shade for ceiling lights, a chandelier, a hanging ornaments for chandeliers, a ceiling hanging light, a shade for ceiling hanging lights, a hanging tool for ceiling lights, a ceiling direct mounting light, a shade for ceiling direct mounting lights, a recessed ceiling light, a louver for ceiling lights, a translucent cover for ceiling lights, a wall light, a shade for wall lights, a wall mounting light, a wall direct mounting light, a shade for wall direct mounting lights, a table lamp, a floor lamp, a shade for table lamps, a street light, a pole for street lights, a glove for street lights, a garden light, a gate lamp, a flashlight, a case for flashlights, a pocket light, a portable electric lamp, an oil lamp, a lantern, a candlestick, a garden lantern, a stone garden lantern, a bactericidal lamp, a projector, a spotlight), heaters and coolers (an electric heater, a coal stove, a gas heater, an oil-heater, a combustion cylinder for stoves, a guard for stoves, a warm air heater, a panel heater, an air conditioner, a room cooler, an outdoor unit for air conditioners, a solar water heater, a feed tank for solar water heaters, a brazier, a fireplace, a charcoal basket, an electric foot warmer, a heater for electric foot warmers, a foot heater, a hot water bottle, a body warmer, a foot warmer, a fan, a ceiling fan, a ventilation fan, a filter for ventilation fans, a hood for ventilation fans, a range hood, a dehumidifier, a humidifier, an air cleaner), kitchen and sanitation goods (a kitchen counter, a sink, a kitchen sink, a garbage can for sinks, a drainboard for sinks, a scrubbing brush rack, a disposer, a range table, a draining rack, a water heater to be attached, a heat exchanger for water heater to be attached, a bath heater, an exhaust pipe for bath heaters, a heat exchanger for bath heaters, a bathtub, a bathtub for infants, a bathtub with washing places, a bathtub lid, a bathtub apron, a showerhead, a washing place for bathrooms, a duckboard for bathrooms, a mat for bathrooms, a soap case, a basin to be attached, a hand washing basin to be attached, a wash dresser, a drain plug for washstands, a front sink, a laundry sink, a water drinking stand, a toilet bowl to be attached, a toilet seat, a cover for toilet seat, an urinal to be attached, a bidet, a waste tank, a septic tank, a water tank for flush toilets), a milk receiving box, a mailbox, a fitting for mailbox ports, a newspaper receiving box, a ladder, a step rung for ladders, a footstool, stepladder, toys (a doll, a ship toy, a vehicles toy, an airplane toy, an intellectual training toy, a sounding toy, a jack-in-the-box, a pacifier, a toy firework, a balloon, a windmill toy), goods for play and pastime (a rocking horse, a jungle gym, a swing, seesaw, a slide, a tricycle for children, a car for children, a three-wheel skate, a skipping rope, bamboo stilts, a battledore, a paddle, a top, a bamboo dragonfly, a cup and a ball, a kite, a ball, a beach ball, a buoy for play quoits, quoits, a yoyo, a pachinko game machine, a slot game machine, a billiard table, a go board, a shogi board, a piece for shogi, sugoroku, a baseball game machine, a mah-jongg table, cards, assembly wooden blocks, an assembly play facility, a puzzle ring, blocks, an insertion play facility, a clipping paper, a folded paper, a painting paper), sporting game goods (a baseball ball, a baseball glove, a baseball mitt, a baseball mask, a baseball bat, a tennis racket, a tennis racket frame, a ping-pong racket, a ping-pong table, a shuttlecock, a badminton racket, a badminton racket frame, a golf club, a shaft for golf clubs, a head for golf clubs, a head cover for golf clubs, a bag for golf clubs, a golf tee, skis, a ski pole, a ring for ski pole, a binding for skis, a case for skis, roller skates, a sled for athletic sports, water skis, a surfboard, a regulator for diving, a snorkel for diving, a fin for diving, an archery, an arrow, a bamboo sword, a trunk protector for kendo, an armguard for kendo, a horizontal bar for exercise, a ice ax, a piton, a barbell, an expander, a hunting gun, an air gun), musical instruments (a keyboard instrument, a wind instrument, a stringed instrument, a percussion instrument, a music synthesizer, a music box, a rhythm generator, a metronome, a pitch pipe, a music stand), goods for hobby and pastime (a birdcage, an insect cage, a doghouse, a collar, a water tank for an appreciation fish), calligraphy tools (a paperweight, an inkstone, an inkstone case, a drawing board, a color box, an engraving cutting edge, a spatula for clay works, a map, a globe, an astronomical chart), stationery (a fountain pen, a mechanical pencil, a ball-point pen, a mechanical pencil with a ball-point pen, a pencil, a brush, a paintbrush, a marking pen, a point protector, a clip for writing materials, a chalk holder, an inkstand, an electric pencil sharpener, a rubber, a plastic sheet, a stamp, a rotary stamp, a stamp pad, an abacus, a drafting board, a drawing table, a tracing stand, a drafting machine, a compass, a divider, a protractor, a template, a set square, a curved ruler, a sealing machine for mail, a mail opener, a paper shredder, a stapler, an electric stapler, a punch for office work, an electric punch for office work, a paper cutter, an eyelet punching, a paper knife, a clip for office work, a pin for office work, a drawing-pin, an eyeleteer, an adhesion tape holder, a filing cabinet, a bookstand, bookends, a pencil case, a letter box, a letter rack, a pen tray, a penholder, a stamp case, a seal box, a telephone chart, a desk calendar, a blackboard, an eraser for blackboards), paper products for office work (a slip, an envelope, a magnetic card, a grid sheet, a letter paper, a mount for albums, a mount for slide films, a tag, a mount for business-cards, a greeting card, a postcard, a picture postcard, a Christmas card, a birthday card, a carbonic paper, a draft, a check, a name card holder, a scrapbook, a pocket diary, a diary, a book of slips, a file, a fitting for file, a binder, a fitting for binder, a hanging folder, a writing sheet scissors, a notebook, a sketchbook, a book of letter papers, an album, a negative cover, a check book, a book jacket, books, a pamphlet, a calendar, a catalog, a poster, a handbill, a bookmark, a coupon, a ticket, a check of footgear, a plate, a nameplate, a price card), packaging goods (a can for packaging, a bottle for packaging, a box for packaging, a slack for packaging, a basket for a packaging, an extrusion tube for packaging, a plate for packaging, an ampoule for packaging, a sprayer for packaging, a bag for packaging, a straw bag, a container for packaging, a spare cap of containers for packaging, a lid of containers for packaging, a crown, a frame for packaging, a wrapping paper, a label, a bookmark for packaging, a blind for packaging, a cover paper, a mount for packaging), advertising tools (an advertising apparatus, a billboard, a bulletin board, an advertising light, an advertising balloon, a road sign, a lightning indicator, a scoreboard, a flag, a triangular pennant, a banner, a flag rod, a merchandise display case, a merchandise display shelf, a merchandise display stand, a refrigerator showcase, a mannequin dummy), transportation machine apparatuses (a crane, a conveyor belt, a screw conveyor, a conveyor chain, a winch, a hoist, a chain block, an elevator, an escalator, a jack, a container, a pallet for transportation, a bomb for transportation, a pulley, a rail for transportation machines), vehicles (an engine, a passenger car, a tram, a seat for rail cars, an automobile, a bus, a truck, a dump truck, a loading platform for a truck, a gate plate for tracks, a tractor, a trailer, a fire engine, a garbage collecting vehicle, a truck crane, a snowmobile, a fork lift truck, a headlight for cars, a taillight for cars, a steering wheel for cars, a console for cars, a shift lever for cars, a rim for cars, a wheel for cars, a hub cap for cars, a wheel cap for cars, a muffler for cars, a bumper for cars, a instrument panel for cars, a lock for cars, a window wiper for cars, an alarm for cars, a heater for cars, an air conditioner for cars, a side mirror for cars, a rearview mirror for cars, a sheet for cars, a radiator grille for cars, a tire chain, a fender for cars, a side visor for cars, a sun visor for cars, a roof carrier for cars, a motorcycle, a motor scooter, a three-wheeled automobile, a headlight for motor bicycles, a taillight for motor bicycles, a shock absorber for motor bicycles, a fuel tank for motor bicycles, a muffler for motor bicycles, a reflector mirror for motor bicycles, a saddle for motor bicycles, a windshield for motor bicycles, a bicycle, a frame of bicycles, a fender for bicycles, a handle for bicycles, a brake lever for bicycles, a caliper brake for bicycles, a pedal for bicycles, a saddle for bicycles, a saddle cover for bicycles, an alarm for bicycles, a headlight for bicycles, a taillight for bicycles, a carrier for bicycles, a stand for bicycles, a lock for bicycles, a front fork for bicycles, a rain shelter for bicycles, a bicycle cart, a cart, a baby carriage, a linear motor car), vessels (a passenger boat, a cargo boat, a motorboat, a sailboat, a mast for sailboats, an oar boat, a canoe, a fishing boat, an outboard motor, an airplane, an airship), electric elements (a dry battery, a storage battery, a solar battery, an electric outlet, a table tap, an attachment plug, a socket for pilot lamps, a socket for electron tubes, a connector for printed wiring, a high frequency coaxial connector, a jack, a plug, an inserting crimp terminal, an contactor, a ground rod, a terminal board, a terminal plate, a knob for electric appliances), electric power distribution (an electric power distribution instrument, a control instrument, an electrical cable, an electric wire installation tool, a rotating electrical machine), communication mechanical apparatuses (a telephone, a public telephone, a cellular telephone, a telephone exchanger, an interphone, a telegraph, a teleprinter, a facsimile, a telephotographic apparatus, a communication relay exchanger, a radio communication device, an antenna, a parabolic antenna, a radio receiving set, a tuner for radios, a tape recorder, a graphic equalizer, an earphone, a headphone, a microphone, a microphone stand, a speaker, a speaker box, a television receiving set, a videotape recorder, a videodisc player, a television camera, a television camera with a videotape recorder, a view finder for television cameras, a cassette tape, a videotape, a record, a compact disc, a video disc, a magneto-optical disc, a digital tape, a digital versatile disc), an electronic calculator, an electronic application mechanical instrument, measuring instruments (a tape measure, a slide calipers, a height gage, a micrometer, a dial gage, a block gauge, a scale, a bathroom scale, a thermometer, a clinical thermometer), clocks (a wristwatch, a watchband, a pocket watch, a stopwatch, a table clock, a wall clock), optical instrument devices (a telescope, a binoculars, a microscope, a magnifying glass, a camera, an over head projector), mechanical devices for office work (an electronic desk calculator, a copying machine, a reader for microfilms, a reader printer for microfilms), automatic vending machines (an automatic vending machine for beverages, an automatic vending machine for frozen-foods, an automatic vending machine for cigarette, an automatic vending machine for tickets, an automatic vending machine for stamps, a money counting machine for automatic vending machines, a money-changing machine, a cash dispenser, an automatic ticket gate), security mechanical instruments (a dustproof mask, a protection mask, a helmet, a life buoy, a life vest, a fire hydrant, a sensor for fires, a rotation alarm lamp, a traffic signal, a reflector for roads, a reflex mirror for roads, a smoke pot, an oil fence), medical-application mechanical instruments (a mechanical instrument for medical-examination facilities, a machinery for physical therapies, a steel instrument for medical treatments, a mechanical instrument for diagnoses, a mechanical instrument for operations, an operative instrument, a mechanical instrument for dentistry, an instrument for rehabilitation), conveniences and tools (a handheld edged tool, a handheld work tool, a portable power tool, a mechanic tool), mechanical instruments for fishing (a fishing tool, a fishing tackle), mechanical instruments for agriculture (a ground-leveling instrument for agriculture, an instrument for cultivating controls, an instrument for harvest adjustments, a straw smoothing machine), mechanical instruments for stockbreeding (a feed grinder, an automatic feeder for stockbreeding, an automatic water supply machine for stockbreeding, a milking machine), a mining machinery, a construction machinery, mechanical apparatuses for chemical processing (a crushing machine, a grinding machine, a separating and removing machine), civil engineering structures (an asphalt road, a concrete road, a wooden road, a bridge beam, a lock bolt, a sluice gate, a sluice gate door, an elevated tank, a gas tank, a steel tower, a telegraph pole, a scaffold metal fitting for telegraph poles, a band for telegraph poles, a caisson, a block, a sheet pile, a joint part of sheet piles, a boundary block between a sidewalk and a roadway, a concrete flat plate for sidewalks, a curbstone block, a lining plate, an expansion joint part for roads, a safety fence for roads, a plate for guardrails, a snowslide prevention fence, a snow protection fence, a block for bank protection, a foot protective block, a block for wave absorbing, a fender for mooring quays, a floating pier, a floating breakwater, an artificial fishing bank, a block for artificial fishing banks, a manhole lid, a ditch lid, a weir for agriculture, a weir column for agriculture), houses (a tent, a greenhouse, a plastic greenhouse, a bathroom, a sauna room, a handrail of windows, a face grille for windows, stairs, a handrail for stairs, a coping for handrails, a coping receiver for handrails, a stanchion for handrails, a vertical bar for handrails, a balcony, a veranda, a fence for verandas, a porch), outdoor equipments (a gate, a gatepost, a door of gates, a wall, a coping for walls, coping receiver for walls, a fence, a vertical bar for fences, an ornament metal fitting for fences, a garden fence, a high place water tank, a panel of high place water tanks, a torii, a telephone booth, a mailbox), constituent members for constructions (a pillar, a beam, a constituent member of a wall, a constituent member of a ceiling, a constituent member of a floor, a constituent member of a roof, a gutter), fittings (a door, a glass door, a sliding paper door, a sliding paper screen, a window screen, a transom window, a partition for buildings, an accordion door, a sliding door, a hinge for fittings, a crescent lock for fittings, a door closer, a pull of a sliding paper door, a lever handle for fittings, a curtain rail, a curtain liner, a curtain stopper, a blanket for curtains, a hook for curtains, a curtain box, a frame for doors, a doorstop, a frame for sliding doors, a double sliding window frame, a pivoted window frame, a center pillar of a shutter, a guide rail of a shutter for buildings, a case of a shutter for buildings), interior or exterior materials for buildings (a roof tile, a concrete block, a tile, a mosaic tile, a floor board, a ceiling board, a wall board, a shingle, a rope, a chain, a bolt, a nut, a wood screw, a split pin, a metal washer, a nail, a rivet, a clamp, a wedge, a hinge, a lock, a key material, a spring, a caster, a wheel for casters, a pipe for piping, a hose, a hose clamp, a vessel body protective cap, a pipe-supporting metal fitting), etc.

In the decomposing process of the present invention, a volatile aromatic compound contained in a vapor phase is decomposed by bringing the volatile aromatic compound into contact with the photocatalyst layer obtained by coating the photocatalyst dispersion liquid on the surface of a substrate.

As for the volatile aromatic compound, for example, benzene, toluene, xylene, methylbenzene, trimethylbenzene, ethylbenzene, styrene, chlorobenzene, dichlorobenzene, trichlorobenzene, cresol, and aniline can be used.

The vapor phase containing the volatile aromatic compound is conventionally the atmosphere.

As for a light for irradiating, a light having a wave length capable of exciting titanium oxide photocatalyst particles and tungsten oxide photocatalyst particles configuring the photocatalyst layer is used. The light can have anyone of ultraviolet rays and visible light. As for a light source, for example, a fluorescent light, a filament lamp, a halogen lamp, a sodium lamp, and sunlight can be used.

The present invention will be described in detail below with examples, but the present invention is not limited to these examples.

In addition, measuring methods in each example are as follows.

### 1. BET specific surface area

BET specific surface areas of the titanium oxide photocatalyst particles and the tungsten oxide photocatalyst particles were measured by a nitrogen adsorbing method using a specific surface area measuring apparatus (MONOSORB produced by Yuasa Ionics Inc.).

### 2. Average dispersed particle diameter (nm)

A particle size distribution of a sample was measured using a submicron particle size distribution measuring apparatus (N4 Plus produced by Beckman Coulter, Inc.), and automatically analyzed with a monodispersion mode by a software attached to this apparatus. The result was made to be an average dispersed particle diameter.

### 3. Crystal structure

An X-ray diffraction spectrum was measured using an X-ray diffraction apparatus (RINT 2000/PC produced by Rigaku Corporation), and a crystal structure was determined from the spectrum.

### 4. Zeta potential of a photocatalyst

Titanium oxide photocatalyst particles or tungsten oxide photocatalyst particles were dispersed in a sodium chloride aqueous solution (having a sodium chloride concentration of 0.01 mol/L) in which a hydrogen ion concentration was adjusted to have pH value of 3.0 by adding hydrogen chloride, and the zeta potential of the solution was measured using a laser zeta potential meter (ELS-6000 produced by Otsuka Electronics Co., Ltd.). A use amount of the sodium chloride aqueous solution was 250000 mass times with respect to a use amount of the titanium oxide photocatalyst particles or the tungsten oxide photocatalyst particles. When the zeta potential was positive, the surface of the photocatalyst was charged positively, and when the zeta potential was negative, the surface was charged negatively.
Reference Example 1 [Preparing of titanium oxide photocatalyst particles and its dispersion liquid]
As the titanium oxide photocatalyst particles, metatitanic acid cake (containing a titanium component of 45% by mass in terms of TiO₂) obtained by hydrolyzing a titanyl sulfate aqueous solution and filtrating it was used.

An oxalic acid aqueous solution was obtained by dissolving oxalic acid dihydration (produced by Wako Pure Chemical Industries, Ltd.) of 158g with water of 1.88kg. A mixture was obtained by adding the metatitanic acid cake of 2.2kg to the oxalic acid aqueous solution and mixing it. The use amount of oxalic acid in this mixture was 0.1 mol with respect to metatitanic acid of 1 mol.

A titanium oxide photocatalyst dispersion liquid was obtained by subjecting the mixture to a dispersion treatment under the following conditions using a medium stirring type dispersing device (ULTRA APEX MILL UAM-1, produced by Kotobuki Engineering & Manufacturing Co., Ltd.).

Dispersion medium: 1.85kg of beads made of zirconia having an outer diameter of 0.05mm
Stirring rate: 12.6 m/sec. at a circumferential speed
Flowing rate: 0.25 L/min
Adding water: Adding pure water of 5kg after 17 minutes from starting a treatment
Treating time: A total of about 90 minutes
The titanium oxide particles of 5 mass parts were obtained in the titanium oxide photocatalyst dispersion liquid of 100 mass parts. The average dispersed particle diameter of the obtained titanium oxide photocatalyst particles in the titanium oxide photocatalyst dispersion liquid was 55 nm. The hydrogen ion concentration had pH value of 1.5. A solid part was obtained by vacuum-drying a part of this titanium oxide photocatalyst dispersion liquid, and the BET specific surface of this solid part was 301 m²/g. The crystal structure of the solid part of the titanium oxide photocatalyst dispersion liquid was anatase. In addition, when the X-ray diffraction spectra of the mixture before the dispersing treatment and the solid part of the titanium oxide photocatalyst dispersion liquid after the dispersing treatment were measured and compared, the change of the crystal structure due to the dispersing treatment was not observed. The zeta potential of the titanium oxide photocatalyst particles in the titanium oxide photocatalyst dispersion liquid was -10.5mV. Reference example 2 [Preparing of tungsten oxide photocatalyst particles and its dispersion liquid]

A mixture was obtained by adding a tungsten oxide powder (having a purity of 99.99%, produced by Kojundo Chemical Laboratory Co., Ltd.) of 1kg to ion-exchanged water of 4kg and mixing it. A tungsten oxide photocatalyst dispersion liquid was obtained by subjecting the mixture to a dispersing treatment under the following conditions using a medium stirring type dispersing device (ULTRA APEX MILL UAM-1, produced by Kotobuki Engineering & Manufacturing Co., Ltd.).

Dispersion medium: 1.85kg of beads made of zirconia having an outer diameter of 0.05mm
Stirring rate: 12.6 m/sec. at a circumferential speed
Flowing rate: 0.25 L/min
Treating time: A total of about 50 minutes

The average dispersed particle diameter of the obtained tungsten oxide photocatalyst particles in the tungsten oxide photocatalyst dispersion liquid was 96 nm. The hydrogen ion concentration had pH value of 2.2. A solid part was obtained by vacuum-drying a part of this dispersion liquid, and the BET specific surface of this solid part was 37 m²/g. In addition, when the X-ray diffraction spectra of the mixture before the dispersing treatment and the solid part of the tungsten oxide photocatalyst dispersion liquid after the dispersing treatment were measured and compared, both the crystal structures were WO₃, and the change of the crystal structure due to the dispersing treatment was not observed. The zeta potential of the tungsten oxide photocatalyst particles in the tungsten oxide photocatalyst dispersion liquid was -25.5mV.

### Example 1

### [Producing of a photocatalyst dispersion liquid]

A photocatalyst dispersion liquid was obtained by mixing the titanium oxide photocatalyst dispersion liquid obtained in the reference example 1 and the tungsten oxide photocatalyst dispersion liquid obtained in the reference example 2 so that the use amount ratio of the titanium oxide photocatalyst particles and the tungsten oxide photocatalyst particles was 1:1 (at mass ratio). A total amount of the titanium oxide photocatalyst particles and the tungsten oxide photocatalyst particles was 5 parts by mass (having a solid part concentration of 5% by mass) in the photocatalyst dispersion liquid of 100 parts by mass. As for this photocatalyst dispersion liquid, the solid-liquid separation was not observed after storing it.

### [Forming of a photocatalyst layer]

The obtained photocatalyst dispersion liquid was dropped at a glass petri dish (having an outer diameter of 70mm, an inner diameter of 66mm, a height of 14mm, and a capacity of about 48mL) (a substrate) so that the dropping amount in terms of the solid part per a unit area of a bottom face was to be 1 g/m², and developed so as to be uniform on the whole bottom face of the perti dish. Then, a photocatalyst layer was formed on the bottom face of the glass petri dish by drying the liquid for one hour under an atmosphere in a dryer at 110°C. A measuring sample was obtained by irradiating an ultraviolet radiation from a black light to the photocatalyst layer for 16 hours so as to have the ultraviolet radiation strength of 2 mW/cm².

### [Measuring of toluene decomposing ability]

The decomposing reaction of toluene was carried out by taking the measuring sample obtained in the above-mentioned example into a gas bag (having an inner capacity of 1L), sealing the bag, making the inside of the gas bag to be a vacuum state, enclosing a mixed gas of 600 mL in which a volume ratio of oxygen and nitrogen was 1:4 in the gas bag, enclosing nitrogen gas containing toluene at a concentration of 1% (at a volume ratio) so that a toluene concentration in the gas bag was to be 20 ppm (at a volume ratio), keeping it in a dark space at a room temperature for 1 hour, and setting the gas bag so that an illuminance near the measuring sample from a commercial white fluorescent light as a light source was to be 1000 lux (measured by an illuminometer "T-10" produced by Konica Minolta Holdings, Inc.). The strength of ultraviolet radiation near the measuring sample was 6.5 µW/cm² (measured by using an ultraviolet intensity meter "UVR-2" produced by Topcon Corporation in which a light receiving part "UD-36" produced by the same corporation to the meter was attached). The gas in the gas bag was sampled every 1.5 hours after irradiating a fluorescent light, the residual concentration of toluene was measured by a gas chromatograph (GC-14A produced by Shimadzu Corporation) so as to calculate a first-order rate constant from the toluene concentration to the irradiation time. The calculated first-order rate constant was 0.314h⁻¹. When the first-order rate constant is greater, the toluene decomposing ability is greater.

### Comparative example 1

A photocatalyst dispersion liquid was prepared by a similar process to that of Example 1 except a commercial titanium oxide photocatalyst dispersion liquid (STS-01, produced by Ishihara Sangyo Kaisha Ltd., containing nitric acid, and having an average dispersed particle diameter of 50nm) was used instead of the titanium oxide photocatalyst dispersion liquid obtained in the reference example 1. The total amount of the titanium oxide photocatalyst particles and the tungsten oxide photocatalyst particles was 5 parts by mass in the photocatalyst dispersion liquid of 100 parts by mass. As for this photocatalyst dispersion liquid, particles were aggregated during storing it, and a solid-liquid separation was generated. In addition, the zeta potential of the titanium oxide particles contained in the titanium oxide dispersion liquid (STS-01) was +40.1mV.

A photocatalyst layer was formed by a similar process to that of Example 1 except the photocatalyst dispersion liquid obtained in this example was used instead of the photocatalyst dispersion liquid obtained in Example 1. When the toluene decomposing ability of the photocatalyst layer was measured, the first-order rate constant was 0.252h⁻¹.

### Comparative example 2

A photocatalyst layer was formed by a similar process to that of Example 1 except the titanium oxide photocatalyst dispersion liquid obtained in the reference example 1 was used independently instead of the photocatalyst dispersion liquid obtained in Example 1. When the toluene decomposing ability of the photocatalyst layer was measured, the first-order rate constant was 0.106h⁻¹.

### Comparative example 3

A photocatalyst layer was formed by a similar process to that of Example 1 except the tungsten oxide photocatalyst dispersion liquid obtained in the reference example 2 was used independently instead of the photocatalyst dispersion liquid obtained in Example 1. When the toluene decomposing ability of the photocatalyst layer was measured, the first-order rate constant was 0.105h⁻¹.

### Example 2

A photocatalyst dispersion liquid was obtained by mixing the titanium oxide photocatalyst dispersion liquid obtained in the reference example 1 and the tungsten oxide photocatalyst dispersion liquid obtained in the reference example 2 so that the use amount ratio of the titanium oxide photocatalyst particles and the tungsten oxide photocatalyst particles was to be 1:1 (at mass ratio), and further adding an aqueous solution of hexachloro platinic acid (H₂PtCl₆) (the content of a platinum component was 0.4% by mass) to the mixture of 100 mass parts. A solid part in the photocatalyst dispersion liquid of 100 parts by mass was 5 parts by mass. A use amount of hexachloro platinic acid was 0.06 parts by mass in terms of the platinum atom with respect to the total use amount of 100 parts by mass of the titanium oxide photocatalyst particles and the tungsten oxide photocatalyst particles. A solid-liquid separation was not observed in this photocatalyst dispersion liquid.

When a photocatalyst layer was formed by a similar process to that of Example 1 except the photocatalyst dispersion liquid obtained in this example was used instead of the photocatalyst dispersion liquid obtained in Example 1, the solid-liquid separation was not observed after storing it, and the first-order rate constant was 0.370h⁻¹.

### Example 3

A yellowish white dispersion liquid was obtained by mixing the titanium oxide photocatalyst dispersion liquid obtained in the reference example 1 and the tungsten oxide photocatalyst dispersion liquid obtained in the reference example 2 so that the use amount ratio of the titanium oxide photocatalyst particles and the tungsten oxide photocatalyst particles was to be 1:1 (at mass ratio), and further adding an aqueous solution of hydrogen tetrachloroaurate (HAuCl₄) (the content of the gold component was 0.47% by mass) and a hydrochloric acid aqueous solution of palladium chloride (obtained by a dissolving palladium chloride (PdCl₂) powder of 0.252g with a mixed solution of a hydrochloric acid aqueous solution of 9.41g having a concentration of 1 mol/L and water of 90.43g) to the mixture of 100 mass parts. The solid part in the dispersion liquid of 100 parts by mass was 5 parts by mass. The use amount of hydrogen tetrachloroaurate was 0.02 parts by mass in terms of the gold atom with respect to the total use amount of 100 parts by mass of the titanium oxide photocatalyst particles and the tungsten oxide photocatalyst particles. The use amount of palladium chloride was 0.01 parts by mass in terms of the palladium atom with respect to the total use amount of 100 parts by mass of the titanium oxide photocatalyst particles and the tungsten oxide photocatalyst particles. A solid-liquid separation was not observed in this dispersion liquid.

A gray photocatalyst dispersion liquid was obtained by transferring the photocatalyst dispersion liquid of 30g to a beaker of 100mL, and irradiating an ultraviolet radiation to the dispersion liquid for 3 hours by an ultrahigh pressure mercury lamp (produced by Ushio Inc., a mercury lamp: USH-250BY, a lamp house: MPL-25101, a lamp power source: HB-2503BY) while stirring it. The hydrogen tetrachloroaurate and the palladium chloride were reduced to gold and palladium respectively. The gold and the palladium were supported on the surfaces of the titanium oxide photocatalyst particles and the tungsten oxide photocatalyst particles. A solid-liquid separation was not observed in this photocatalyst dispersion liquid.

When a photocatalyst layer was formed by a similar process to that of Example 1 except the photocatalyst dispersion liquid obtained in this example was used instead of the photocatalyst dispersion liquid obtained in Example 1, the solid-liquid separation was not observed after storing it, and the first-order rate constant was 0.374h⁻¹.

### Example 4

By coating and drying each photocatalyst dispersion liquid obtained in Examples 1 to 3 on a surface of a ceiling plate constructing a ceiling, a photocatalyst layer could be formed on the surface of the ceiling plate. By irradiating light from an interior lighting, the concentration of a volatile aromatic compound such as toluene contained in the indoor air could be reduced.

### Example 5

By coating and drying each photocatalyst dispersion liquid obtained in Examples 1 to 3 on a tile disposed on a wall surface in the indoor space, a photocatalyst layer could be formed on the surface of the tile. By irradiating light from an interior lighting, the concentration of a volatile aromatic compound such as toluene contained in the indoor air could be reduced.

### Example 6

By coating and drying each photocatalyst dispersion liquid obtained in Examples 1 to 3 on an indoor-side surface of a window glass, a photocatalyst layer could be formed on the surface of the window glass. By irradiating light from an interior lighting, the concentration of a volatile aromatic compound such as toluene contained in the indoor air could be reduced.

### Example 7

By coating and drying each photocatalyst dispersion liquid obtained in Examples 1 to 3 on a wallpaper, a photocatalyst layer could be formed on the surface of the wallpaper. When this wallpaper was disposed on a wall surface in the indoor space, the concentration of a volatile aromatic compound such as toluene contained in the indoor air could be reduced by irradiating light from an interior lighting.

### Example 8

By coating and drying each photocatalyst dispersion liquid obtained in Examples 1 to 3 on surfaces of automobile upholsteries such as an automobile instrument panel, an automobile sheet, an automobile ceiling material, and the like, a photocatalyst layer could be formed on the surfaces of the automobile upholsteries. By irradiating light from a lighting inside a vehicle, the concentration of a volatile aromatic compound such as toluene contained in the air in an inside space of a vehicle could be reduced.

### Example 9

By coating and drying each photocatalyst dispersion liquid obtained in Examples 1 to 3 on a floor in the indoor space, a photocatalyst layer could be formed on the surface of the floor. By irradiating light from an interior lighting, the concentration of a volatile aromatic compound such as toluene contained in the indoor air could be reduced.

## Claims

1. A process for decomposing a volatile aromatic compound in the vapor phase, which process comprises:
a step of bringing the volatile aromatic compound into contact, under light irradiation, with a photocatalyst layer formed by applying a photocatalyst dispersion liquid to a substrate,
wherein the photocatalyst dispersion liquid comprises titanium oxide photocatalyst particles and tungsten oxide photocatalyst particles dispersed in a dispersion medium, and the surfaces of the titanium oxide photocatalyst particles are charged in the same polarity as the surfaces of the tungsten oxide photocatalyst particles.

2. A process according to claim 1, wherein the photocatalyst dispersion liquid contains an electron-withdrawing substance or a precursor thereof.

3. A process according to claim 2, wherein the electron-withdrawing substance or its precursor is a particle of at least one kind of metal selected from a group consisting of Cu, Pt, Au, Pd, Ag, Fe, Nb, Ru, Ir, Rh, and Co, or a compound thereof.

4. A process according to claim 2 or 3, wherein the amount of the electron-withdrawing substance or its precursor is from 0.005 to 0.6 parts by mass in terms of the metal atom with respect to the total amount of 100 parts by mass of the titanium oxide photocatalyst particles and the tungsten oxide photocatalyst particles.

5. A process according to any one of claims 1 to 4, wherein the average dispersed particle diameter of the titanium oxide photocatalyst particles is from 20nm to 150nm.

6. A process according to any one of claims 1 to 5, wherein the BET specific surface of the titanium oxide photocatalyst particles is from 100 m²/g to 500 m²/g.

7. A process according to any one of claims 1 to 6, wherein the average dispersed particle diameter of the tungsten oxide photocatalyst particles is from 50nm to 200nm.

8. A process according to any one of claims 1 to 7, wherein the BET specific surface of the tungsten oxide photocatalyst particles is from 5 m²/g to 100 m²/g.

9. A process according to any one of claims 1 to 8, wherein the mass ratio of the titanium oxide photocatalyst particles to the tungsten oxide photocatalyst particles is from 4:1 to 1:8.

10. A process according to any one of claims 1 to 9, wherein the mass of the dispersion medium is from 5 times to 200 times with respect to the total mass of the titanium oxide photocatalyst particles and the tungsten oxide photocatalyst particles.

11. A process according to any one of claims 1 to 10, wherein the photocatalyst dispersion liquid has a pH of from 0.5 to 8.0.

12. A process according to any one of claims 1 to 11, wherein the volatile aromatic compound is at least one compound selected from a group consisting of benzene, toluene, xylene, methylbenzene, trimethylbenzene, ethylbenzene, styrene, chlorobenzene, dichlorobenzene, trichlorobenzene, cresol, and aniline.

13. A process according to any one of claims 1 to 12, wherein the vapor phase containing the volatile aromatic compound is the atmosphere.

14. A process according to any one of claims 1 to 13, wherein a light source for irradiating light is selected from a group consisting of a fluorescent light, a filament lamp, a halogen lamp, a sodium lamp, and sunlight.
